# EUROPEAN PATENT SPECIFICATION

(11) **EP 1 512 686 B1**
(45) Date of publication and mention of the grant of the patent: **06.09.2006**
(21) Application number: 04022441.2
(22) Date of filing: 11.12.1998
(51) Int. Cl.: C07D 471/14, A61K 31/44, A61K 31/505, C07D 401/06, C07D 471/04

(54) **IMIDAZONAPHTHYRIDINES AND THEIR USE IN INDUCING CYTOKINE BIOSYNTHESIS**
IMIDAZONAPHTHYRIDINE UND IHRE VERWENDUNG ZUR INDUZIERUNG DER BIOSYNTHESE VON CYTOKIN
IMIDAZONAPHTHYRIDINES ET LEUR UTILISATION DANS L'ACTIVATION DE LA BIOSYNTHESE DE CYTOKINES

(30) Priority: 11.12.1997 US 69276 P
(43) Date of publication of application: 09.03.2005
(62) Divisional of application: 98963888.7
(73) Proprietor: MINNESOTA MINING AND MANUFACTURING COMPANY, St. Paul, Minnesota 55133-3427 (US)
(72) Inventor: Lindstrom, Kyle J., Saint Paul Minnesota 55133-3427 (US)
(74) Representative: Vossius & Partner

(56) References cited:
- WO-A-93/09119
- US-A- 4 689 338
- US-A- 4 929 624

## Description

This invention relates to the imidazonaphthyridine compound 1-(2-methylpropyl)-1H-imidazo[4,5-c][1,5]naphthyridin-4-amine or pharmaceutically acceptable salts thereof. This invention additionally relates to pharmaceutical compositions containing the above imidazonaphthyridine compounds. A further aspect of this invention relates to the use of these compounds as immunomodulators and for inducing cytokine biosynthesis in animals.

The first reliable report on the 1*H*-imidazo[4,5-*c*]quinoline ring system, Backman et al., J. Org. Chem. 15, 1278-1284 (1950) describes the synthesis of 1-(6-methoxy-8-quinolinyl)-2-methyl-1*H*-imidazo[4,5-*c*]quinoline for possible use as an antimalarial agent. Subsequently, syntheses of various substituted 1*H*-imidazo[4,5-*c*] quinolines were reported. For example, Jain et al., J. Med. Chem. 11, pp. 87-92 (1968), synthesized the compound 1-[2-(4-piperidyl)ethyl]-1*H*-imidazo[4,5-*c*]quinoline as a possible anticonvulsant and cardiovascular agent. Also, Baranov et al., Chem. Abs. 85, 94362 (1976), have reported several 2-oxoimidazo[4,5-*c*]quinolines, and Berenyi et al., J. Heterocyclic Chem. 18, 1537-1540 (1981), have reported certain 2-oxoimidazo[4,5-c]quinolines.

Certain 1*H*-imidazo[4,5-*c*]quinolin-4-amines and 1- and 2-substituted derivatives thereof were later found to be useful as antiviral agents, bronchodilators and immunomodulators. These are described in, *inter alia,* U.S. Patent Nos. 4,689,338; 4,698,348; 4,929,624; 5,037,986; 5,268,376; 5,346,905; and 5,389,640, all of which arc incorporated herein by reference. Although there continues to be interest in the imidazoquinoline ring system, as seen for example in WO 98/30562, there is a continuing need for compounds that have the ability to modulate the immune response, by induction of cytokine biosynthesis or other mechanisms.

The present invention relates to the compound 1-(2-methylpropyl)-1H-imidazo[4,5-c][1,5]naphthyridin-4-amine or a pharmaceutically acceptable salt thereof. Furthermore, the present invention provides a pharmaceutical composition comprising a pharmaceutically effective amount of the above compound or salt and a pharmaceutically acceptable carrier. Another embodiment of the present invention relates to the above compound or salt for use in a method of inducing cytokine biosynthesis in an animal. In addition, the present invention relates to the use of the above compound or salt for preparing a medicament for inducing cytokine biosynthesis in an animal. A further embodiment of the present invention relates to the above compound or salt for use in a method of treating a viral infection in an animal. Moreover, the present invention provides the use of the above compound or salt for preparing a medicament for treating a viral infection in an animal.

The compounds of the present invention are useful as immune response modifiers due to their ability to induce cytokine biosynthesis and otherwise modulate the: immune reponse when administered to animals. This ability makes the compounds useful in the treatment of a variety of conditions, e.g. viral diseases and tumors that are responsive to such changes in the immune response.

The present specification describes compounds of Formula I: 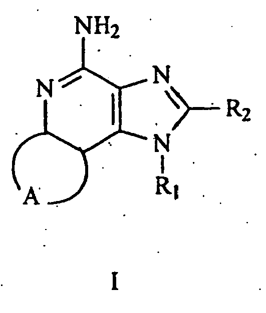 wherein
**A** is =N-CR=CR-CR=; =CR-N=CR-CR=; =CR-CR=N-CR=; or =CR-CR=CR-N=;
**R**_{**1**} is selected from the group consisting of:
- hydrogen;
- C₁₋₂₀ alkyl or C₂₋₂₀ alkenyl that is unsubstituted or substituted by one or more substituents selected from the group consisting of:
   -aryl;
   -heteroaryl;
   -heterocyclyl;
   -O-C₁₋₂₀ alkyl,
   -O-(C₁₋₂₀alkyl)₀₋₁-aryl;
   -O-(C₁₋₂₀alkyl)₀₋₁-heteroaryl;
   -O-(C₁₋₂₀alkyl)₀₋₁-heterocyclyl;
   -C₁₋₂₀ alkoxycarbonyl;
   -S(O)₀₋₂ -C₁₋₂₀ alkyl;
   -S(O)₀₋₂-(C₁₋₂₀ alkyl)₀₋₁-aryl;
   -S(O)₀₋₂-(C₁₋₂₀ alkyl)₀₋₁-heteroaryl;
   -S(O)₀₋₂-(C₁₋₂₀ alkyl)₀₋₁-heterocyclyl;
   -N(R₃)₂;
   -N₃;
   oxo;
   -halogen;
   -NO₂;
   -OH; and
   -SH; and
- C₁₋₂₀ alkyl-NR₃-Q-X-R₄ or -C₂₋₂₀ alkenyl-NR₃-Q-X-R₄ wherein **Q** is -CO- or -SO₂-; **X** is a bond, -O- or -NR₃- and **R**_{**4**} is aryl; heteroaryl; heterocyclyl; or -C₁₋₂₀ alkyl or C₂₋₂₀ alkenyl that is unsubstituted or substituted by one or more substituents selected from the group consisting of:
   -aryl;
   -heteroaryl;
   heterocyclyl;
   -O-C₁₋₂₀ alkyl,
   -O-(C₁₋₂₀alkyl)₀₋₁-aryl;
   -O-(C₁₋₂₀alkyl)₀₋₁-heteroaryl;
   -O-(C₁₋₂₀alkyl)₀₋₁-heterocyclyl;
   -C₁₋₂₀ alkoxycarbonyl;
   -S(O)₀₋₂-C₁₋₂₀ alkyl;
   -S(O)₀₋₂-(C₁₋₂₀alkyl)₀₋₁-aryl;
   -S(O)₀₋₂-(C₁₋₂₀ alkyl)₀₋₁-heteroaryl;
   -S(O)₀₋₂ -(C₁₋₂₀ alkyl)₀₋₁-heterocyclyl;
   -N(R₃)₂;
   -NR₃-CO-O-C₁₋₂alkyl;
   -N₃;
   oxo;
   -halogen;
   -NO₂;
   -OH; and
   -SH; or **R**_{**4**} is 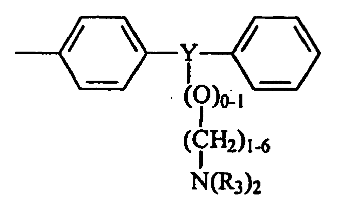
   wherein **Y** is -N- or -CR-;
**R**_{**2**} is selected from the group consisting of:
-hydrogen;
-C₁₋₁₀ o alkyl;
-C₂₋₁₀ alkenyl;
-aryl;
-C₁₋₁₀alkyl-O-C₁₋₁₀-alkyl;
-C₁₋₁₀ alkyl-O-C₂₋₁₀ alkenyl; and
-C₁₋₁₀ alkyl or C₂₋₁₀ alkenyl substituted by one or more substituents selected from the group consisting of:
   -OH;
   -halogen;
   -N(R₃)₂;
   -CO-N(R₃)₂;
   -CO-C₁₋₁₀ alkyl;
   -N₃;
   -aryl;
   -heteroaryl;
   -heterocyclyl;
   -CO-aryl; and
   -CO-heteroaryl;
each **R**_{**3**} is independently selected from the group consisting of hydrogen and C₁₋₁₀ alkyl; and
each **R** is independently selected from the group consisting of hydrogen, C₁₋₁₀ alkyl, C₁₋₁₀ alkoxy, halogen and trifluoromethyl,
or a pharmaceutically acceptable salt thereof.

As used herein, the terms "alkyl", "alkenyl", and the prefix "-alk" are inclusive of both straight chain and branched chain groups and of cyclic groups, i.e. cycloalkyl and cycloalkenyl. These cyclic groups can be monocyclic or polycyclic and preferably have from 3 to 10 ring carbon atoms. Exemplary cyclic groups include cyclopropyl, cyclopentyl, cyclohexyl and adamantyl.

The term "aryl" as used herein includes carbocyclic aromatic rings or ring systems. Examples of aryl groups include phenyl, naphthyl, biphenyl, fluorenyl and indenyl. The term "heteroaryl" includes aromatic rings or ring systems that contain at least one ring hetero atom (e.g., O, S, N). Suitable heteroaryl groups include furyl, thienyl, pyridyl, quinolinyl, tetrazolyl, imidazo, and so on.

"Heterocyclyl" includes non-aromatic rings or ring systems that contain at least one ring hetero atom (e.g., O, S, N). Exemplary heterocyclic groups include pyrrolidinyl, tetrahydrofuranyl, morpholinyl, thiazolidinyl, and imidazolidinyl.

The Aryl, heteroaryl and heterocyclyl groups may be unsubstituted or substituted by one or more substituents selected from the group consisting of C₁₋₂₀ alkyl, hydroxy, halogen, N(R₃)₂, NO₂, C₁₋₂₀ alkoxy, C₁₋₂₀alkylthio, trihalomethyl, C₁₋₂₀ acyl, arylcarbonyl, heteroarylcarbonyl, (C₁₋₁₀alkyl)₀₋₁-aryl, (C₁₋₁₀alkyl)₀₋₁-heteroaryl, nitrile, C₁₋₂₀ alkoxycarbonyl, oxo, arylalkyl wherein the alkyl group has from 1 to 10 carbon atoms, and heteroarylalkyl wherein the alkyl group has from 1 to 10 carbon atoms.

The invention is inclusive of the compound 1-(2-methylpropyl)-1H-imidazo[4,5-c][1,5]naphthyridin-4-amine in any of its pharmaceutically acceptable forms, including isomers such as enantiomers, salts, solvates, polymorphs, and the like.

### Preparation of the Compounds

Compounds of Formula I wherein A is =CR-CR=CR-N= and R, R₁ and R₂ are as defined above can be prepared according to Reaction Scheme III.

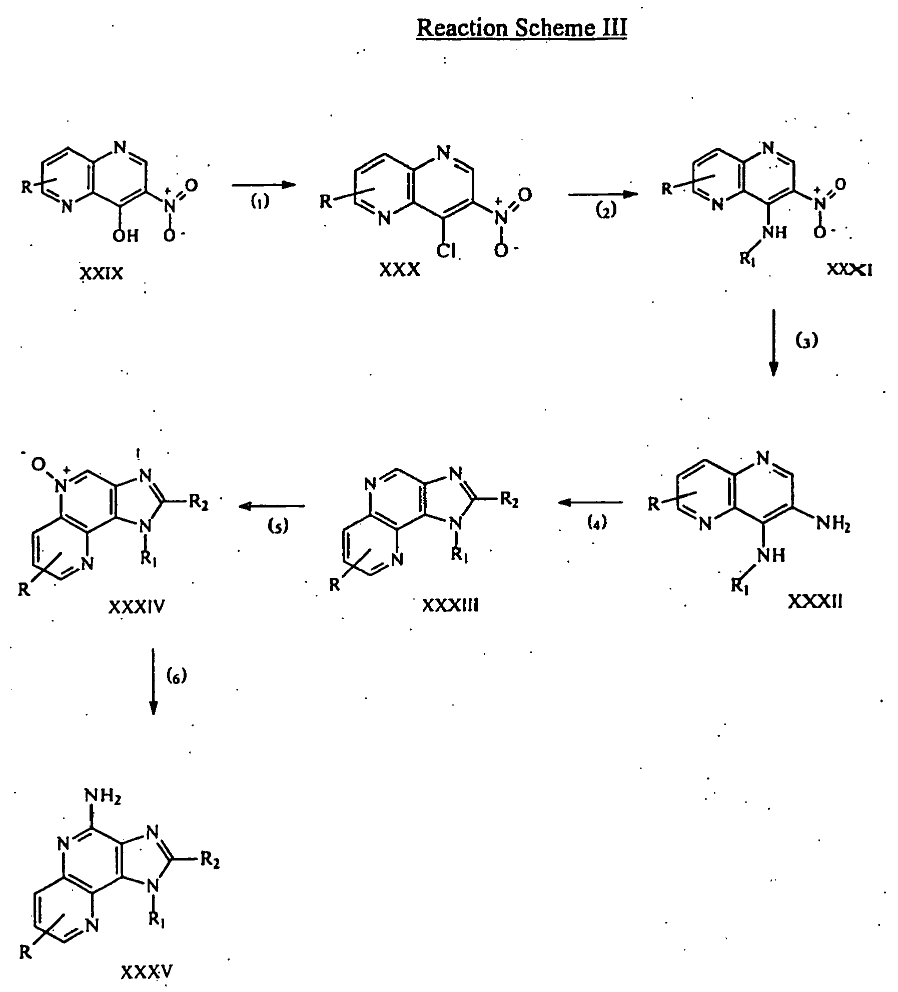

In step (1) of Reaction Scheme III a 3-nitro[1,5]naphthyridin-4-ol of Formula XXIX is chlorinated using a suitable chlorinating agent such as phosphorus oxychloride to provide a 4-chloro-3-nitro[1,5]naphthyridine of Formula XXX. The reaction can be carried out by reacting a compound of Formula XXIX with phosphorus oxychloride in a suitable solvent such as N,N-dimethylformamide with mild heating (~55° C). The compound may be isolated by conventional methods or it can be carried on without isolation as described below in connection with step (2). The compound of Formula XXIX where R is hydrogen is known and its preparation has been disclosed in Hart, Journal of the Chemical Society pp. 212-214, (1956).

In step (2) of Reaction Scheme III a 4-chloro-3-nitro[1,5]naphthyridine of Formula XXX is reacted with an amine of Formula R₁NH₂ where R₁ is as defined above to provide a 3-nitro[1,5]naphthyridin-4-amine of Formula XXXI. The reaction can be carried out by adding water then excess amine to the reaction mixture resulting from step (1) then heating on a steam bath. The reaction can also be carried out by adding excess amine to a solution of a compound of Formula XXX in a suitable solvent such as dichloromethane and optionally heating. The compound of Formula XXXI where R₁ is hydrogen is known and its preparation has been disclosed in Wozniak et al, J. R. Neth. Chem. Soc. 102 (12), pp. 511-13 (1983).

In step (3) of Reaction Scheme III a 3-nitro[1,5]naphthyridin-4-amine of Formula XXXI is reduced to provide a [1,5]naphthyridine-3,4-diamine of Formula XXXII. Preferably, the reduction is carried out using a conventional heterogeneous hydrogenation catalyst such as platinum on carbon or palladium on carbon. The reaction can conveniently be carried out on a Parr apparatus in a suitable solvent such as ethyl acetate.

In step (4) of Reaction Scheme III a compound of Formula XXXII is reacted with a carboxylic acid or an equivalent thereof to provide a *1H-*imidazo[4,5-*c*][1,5]naphthyridine of Formula XXXIII. Suitable equivalents to carboxylic acid include acid halides, orthoesters, and 1,1-dialkoxyalkyl alkanoates. The carboxylic acid or equivalent is selected such that it will provide the desired R₂ substituent in a compound of Formula XXXIII. For example, diethoxymethylacetate will provide a compound where R₂ is hydrogen and trimethylorthovalerate will provide a compound where R₂ is butyl. The reaction can be run in the absence of solvent, in a carboxylic acid such as acetic acid, or in an inert solvent in the presence of an acid. The reaction is run with sufficient heating to drive off any alcohol or water formed as a byproduct of the reaction.

Alternatively, step (4) may be carried out by (i) reacting a compound of Formula XXXII with an acylating agent; and then (ii) cyclizing the product. Part (i) involves reacting a compound of Formula XXXII with an acyl halide of formula R₂C(O)X wherein R₂ is as defined above and X is chloro or bromo. The reaction can be carried out by adding the acyl halide in a controlled fashion (e.g. dropwise) to a solution of a compound of Formula XXXII in a suitable solvent such as dichloromethane at a reduced temperature (e.g., 0°C). The resulting amide intermediate can be isolated by removal of the solvent. Part (ii) involves cyclizing the product of part (i) by reacting it with methanolic ammonia at an elevated temperature (e.g. 150°C) and pressure.

In step (5) of Reaction Scheme III a compound of Formula XXXIII is oxidized to provide a *1H-*imidazo[4,5-*c*][1,5]naphthyridine-5N-oxide of Formula XXXIV using a conventional oxidizing agent that is capable of forming N-oxides. Preferred reaction conditions involve reacting a solution of a compound of Formula XXXIII in chloroform with 3-chloroperoxybenzoic acid at ambient conditions.

In step (6) of Reaction Scheme III a compound of Formula XXXIV is ami nated to provide a *1H*-imidazo[4,5-*c*][1,5]naphthyridin-4-amine of Formula XXXV which is a subgenus of Formula 1. Step (6) involves (i) reacting a compound of formula XXXIV with an acylating agent; and then (ii) reacting the product with an aminating agent. Part (i) of step (6) involves reacting an N-oxide with an acylating agent. Suitable acylating agents include alkyl- or arylsulfonyl chlorides (e.g., benzenesulfonyl chloride, methanesulfonyl choride, p-toluenesulfonyl chloride). Arylsulfonyl chlorides are preferred. p-Toluenesulfonyl chloride is most preferred. Part (ii) of step (6) involves reacting the product of part (i) with an excess of an aminating agent. Suitable aminating agents include ammonia (e.g., in the form of ammonium hydroxide) and ammonium salts (e.g., ammonium carbonate, ammonium bicarbonate, ammonium phosphate). Ammonium hydroxide is preferred. The reaction is preferably carried out by dissolving the N-oxide of Formula XXXIV in an inert solvent such as dichloromethane, adding the aminating agent to the solution, and then adding the acylating agent. Preferred conditions involve cooling to about 0°C to about 5°C during the addition of the acylating agent. The product or a pharmaceutically acceptable salt thereof can be isolated using conventional methods.

Alternatively step (6) may be carried out by (i) reacting a compound of Formula XXXIV with an isocyanate; and then (ii) hydrolyzing the product. Part (i) involves reacting the N-oxide with an isocyanate wherein the isocyanato group is bonded to a carbonyl group. Preferred isocyanates include trichloroacetyl isocyanate and aroyl isocyanates such as benzoyl isocyanate. The reaction of the isocyanate with the N-oxide is carried out under substantially anhydrous conditions by adding the isocyanate to a solution of the N-oxide in an inert solvent such as dichloromethane. The resulting product can be isolated by removal of the solvent. Part (ii) involves hydrolysis of the product from part (i). The reaction can be carried out by conventional methods such as heating in the presence of water or a lower alkanol optionally in the presence of a catalyst such as an alkali metal hydroxide or lower alkoxide.

Certain functional groups recited in connection with R₁ and R₂ may be incompatible with some of the reagents of Reaction Scheme III. Compounds containing such functional groups can be prepared by those skilled in the art using well known methods of functional group protection and manipulation. For example, amine groups may be protected when necessary by derivatizing with di-*tert*-butyl dicarbonate.

Some compounds of Formula I containing certain functional groups may be readily prepared from other compounds of Formula I. For example, compounds wherein the R₁ substituent contains an amide group may conveniently be prepared by reacting an acid chloride with a compound of Formula I wherein the R₁ substituent contains a primary amine. Likewise, compounds wherein the R₁ substituent contains a urea group may be prepared by reacting an isocyanate with a compound of Formula I wherein the R₁ substituent contains a primary amine. Further, compounds wherein the R₁ substituent contains a carbamate group may be prepared by reacting a chloroformate with a compound of Formula I wherein the R₁ substituent contains a primary amine.

### Pharmaceutical Compositions and Biological Activity

Pharmaceutical compositions of the invention contain a therapeutically effective amount of the compound 1-(2-methylpropyl)-1H-imidazo[4,5-c][1,5]naphthyridin-4-amine or a pharmaceutically acceptable salt thereof. in combination with a pharmaceutically acceptable carrier. As used herein, the term "a therapeutically effective amount" means an amount of the compound sufficient to induce a therapeutic effect, such as cytokine induction or antiviral activity. Although the exact amount of active compound used in a pharmaceutical composition of the invention will vary according to factors known to those of skill in the art, such as the physical and chemical nature of the compound as well as the nature of the carrier and the intended dosing regimen, it is anticipated that the compositions of the invention will contain sufficient active ingredient to provide a dose of about 100ng/kg to about 50mg/kg, preferably about 10µg/kg to about 5mg/kg of the compound to the subject. Any of the conventional dosage forms m ay be used, such as tablets, lozenges, parenteral formulations, syrups, creams, ointments, aerosol formulations, transdermal patches, transmucosal patches and so on.

The compounds of the invention have been shown to induce the production of certain cytokines in experiments performed according to the Test Method set forth below. This ability indicates that the compounds are useful as immune response modifiers that can modulate the immune response in a number of different ways, rendering them useful in the treatment of a variety of disorders.

Cytokines that are induced by the administration of compounds according to the invention generally include interferon (IFN) and tumor necrosis factor (TNF) as well as certain interleukins (IL). In particular, the compounds induce IFN-α, TNF-α, IL-1, 6, 10 and 12, and a variety of other cytokines. Among other effects, cytokines inhibit virus production and tumor cell growth, making the compounds useful in the treatment of tumors and viral diseases.

In addition to the ability to induce the production of cytokines, the compounds affect other aspects of the innate immune response. For example, natural killer cell activity may be stimulated, an effect that may be due to cytokine induction. The compounds may also activate macrophages, which in turn stimulates secretion of nitric oxide and the production of additional cytokines. Further, the compounds may cause proliferation and differentiation of B-lymphocytes.

Compounds of the invention also have an effect on the acquired immune response. For example, although there is not believed to be any direct effect on T cells or direct induction ofT cell cytokines, the production of the T helper type 1 (Th1 cytokine IFN-γ is induced indirectly and the production of the Th2 cytokine IL-5 is inhibited upon administration of the compounds. This activity means that the compounds are useful in the treatment ofdiseases where upregulation of the Th1 response and/or downregulation of the Th2 response is desired. In view of the ability of the above compounds to inhibit T-helper-type 2 immune response, the compounds are expected to be useful in the treatment of atopy, e.g., atopic dermatitis, asthma, allergy, allergic rhinitis; as a vaccine adjuvant for cell mediated immunity; and possibly as a treatment for recurrent fungal diseases and chlamydia.

The immune response modifying effects of the compounds make them use ful in the treatment of a wide variety of conditions. Because of their ability to induce cytokines such as IFN-α and TNF-α, the compounds are particularly useful in the treatment of viral diseases and tumors. This immunomodulating activity suggests that compounds of the invention are useful in treating diseases such as, but not limited to viral diseases e. g. genital warts, common warts, plantar warts, Hepatitis B, Hepatitis C, Herpes Simp lex Type I and Type II, molluscum contagiosm, HIV, CMV, VZV, cervical intraepithelial neoplasia, human papillomavirus and associated neoplasias; fungal diseases, e.g. candida, aspergillus, cryptococcal meningitis; neoplastic diseases, e.g., basal cell carcinoma, hairy cell leukemia, Kaposi's sarcoma, renal cell carcinoma, squamous cell carcinoma, myelogenous leukemia, multiple myeloma, melanoma, non-Hodgkin's lymphoma, cutaneous T-cell lymphoma, and other cancers; parasitic diseases, e.g. pneumocystis camii, cryptosporidiosis, histoplasmosis, toxoplasmosis, trypanosome infection, leishmaniasis; bacterial infections, e.g., tuberculosis, mycobacterium avium. Additional diseases or conditions that can be treated using the compounds of the invention include eczema, eosinophilia, essential thrombocythaemia, leprosy, multiple sclerosis, Ommen's syndrome, rheumatoid arthritis, systemic lupus erythematosis, discoid lupus, Bowen's disease and Bowenoid papulosis.

Accordingly, the present specification describes a method of inducing cytokine biosynthesis in an animal comprising administering an effective amount of the above compound to the animal. An amount of a compound effective to induce cytokine biosynthesis is an amount sufficient to cause one or more cell types, such as monocytes, macrophages, dendritic cells and B-cells to produce an amount of one or more cytokines such as, for example, INF-α, TNF-α, IL-1,6,10 and 12 that is increased over the background level of such cytokines. The precise amount will vary according to factors known in the art but is expected to be a dose of about 100ng/kg to about 50mg/kg, preferably about 10µg/kg to about 5mg/kg. The present specification further describes a method of treating a viral infection in an animal comprising administering an effective amount of the above compound to the animal. An amount effective to treat or inhibit a viral infection is an amount that will cause a reduction in one or more of the manifestations of viral infection, such as viral lesions, viral load, rate of virus production, and mortality as compared to untreated control animals. The precise amount will vary according to factors known in the art but is expected to be a dose of 100ng/kg to about 50mg/kg, preferably about 10µg/kg to about 5mg/kg.

The invention is further described by the following examples, which are provided for illustration only and are not intended to be limiting in any way.

### Example 1 (Reference example)

### Compound of Formula XXXI

### N⁴-(2-Methylpropyl)-3-nitro[1,5]naphthyridin-4-amine

Phosphorous oxychloride (0.6 mL, 6.44 mmol) was reacted with N,N-dimethylformamide then added to a solution of 3-nitro[1,5]naphthyridin-4-ol (1.0 g, 5.23 mmol) in N,N-dimethylformamide (20 mL). The reaction mixture was warmed using a jacketed flask with refluxing acetone as a heat source. After 3 hours the reaction mixture was poured into ice water, isobutylamine (2.0 mL, 20.1 mmol) was added and the mixture was heated on a steam bath. After several hours the reaction mixture was cooled to ambient temperature, filtered and washed with water. The aqueous layer was extracted with dichloromethane. The dichloromethane extract was washed with aqueous sodium bicarbonate, washed with water, dried over magnesium filtrate then loaded onto a layer of silica gel. The silica gel was eluted initially with dichloromethane to remove an impurity then with 5% methanol in dichloromethane to recover the product. The eluant was concentrated to dryness to provide N⁴-(2-methylpropyl)-3-nitro[1,5]naphthyridin-4-amine as a solid, m.p., 97-99°C.

### Example 2 (Reference example)

### Compound of Formula XXXIII

### 1-(2-Methylpropyl)-1H-imidazo[4,5-c][1,5[naphthyridine

### Part A:

A catalytic amount of 5% platinum on carbon was added to a solution of N⁴-(2-methylpropyl)-3-nitro[1,5]naphthyridin-4-amine (1.0 g, 4.1 mmol) in ethyl acetate (50 mL). The reaction mixture was reduced on a Parr apparatus at 50 psi (3.5 Kg/cm²) hydrogen for four hours. The reaction mixture was filtered to remove the catalyst and the filtrate was concentrated under vacuum to provide N⁴-(2-methylpropyl)[1,5]naphthyridin-3,4-diamine as a crude solid.

### Part B:

The crude solid from Part A was combined with diethoxymethylacetate (2 mL) then heated on a steam bath overnight. The reaction mixture was taken up in dichloromethane, washed with water, dried over magnesium sulfate then filtered through a layer of silica gel. The silica gel was eluted with dichloromethane to remove excess diethoxymethylacetate then with 5% methanol in dichloromethane to recover the product. The eluant was concentrated to provide an oil which was purified by flash chromatography (silica gel eluting with 50% ethyl acetate/hexane then with ethyl acetate) to provide 0.25 g of 1-(2-methylpropyl)-*1H*-imidazo[4,5-*c*][1,5]naphthyridine as a solid m.p. 82-84°C. Analysis: Calculated for C₁₃H₁₄N₄: %C, 69.00; %H, 6.24; %N, 24.76; Found: %C, 68.79; %H, 6.44; %N, 24.73.

### Example 3 (Reference example)

### Compound of Formula XXXIV

### 1-(2-Methylpropyl)-1H-imidazo[4,5-c][1,5]naphthyridine-5N-oxide

3-Chloroperoxybenzoic acid (3.7 g of 50%) was added in small portions over a period of 30 minutes to a solution of 1-(2-methylpropyl)-1*H*-imidazo[4,5-*c*][1,5]naphthyridine (1.5 g) in chloroform at ambient temperature. After 3 hours the reaction mixture was diluted with chloroform, washed twice with 2.0 M sodium hydroxide and once with water, dried over magnesium sulfate then concentrated under vacuum. The residue was recrystallized from ethyl acetate/hexane to provide 1.2 g of 1-(2-methylpropyl)-*1H*-imidazo[4,5-*c*][1,5]naphthyridine-5N-oxide as a solid, m.p. 183-185°C. Analysis: Calculated for C₁₃H₁₄N₄O: %C, 64.45; %H, 5.82; %N, 23.12; Found: %C, 64.15; %H, 5.92; %N, 23.02.

### Example 4

### Compound of Formula I

### 1-(2-Methylpropyl)-1H-imidazo[4,5-c][1,5]naphthyridin-4-amine

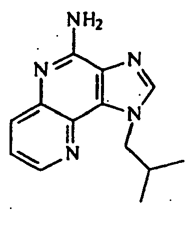

Ammonium hydroxide (10 mL) was added to a solution of 1-(2-methylpropyl)-*1H-*imidazo[4,5-*c*][1,5]naphthyridine-5N-oxide (0.6 g) in dichloromethane (30mL). The reaction mixture was cooled in an ice bath then tosyl chloride (0.5 g) in dichloromethane was added while the reaction was being rapidly stirred. The reaction mixture was stirred at ambient temperature overnight. The dichloromethane layer was separated, washed with aqueous sodium bicarbonate, dried over magnesium sulfate then concentrated under vacuum. The residue was recrystallized from ethyl acetate/hexane to provide 0.2 g of 1-(2-methylpropyl)-*1H-*imidazo[4,5-*c*][1,5]naphthyridin-4-amine as a solid, m.p. 230-231.5°C. Analysis:Calculated for C₁₃H₁₅N₅: %C, 64.71; %H, 6.27; %N, 29.02; Found: %C, 64.70; %H, 6.01; %N, 29.08.

### TEST METHODS

### CYTOKINE INDUCTION IN HUMAN CELLS

An in vitro human blood cell system was used to assess cytokine induction by compounds of the invention. Activity is based on the measurement of interferon and tumor necrosis factor (∝) (IFN and TNF, respectively) secreted into culture media as described by Testerman et. al. In "Cytokine Induction by the Immunomodulators Imiquimod and S-27609", Journal of Leukocyte Biology, **58**, 365-372 (September, 1995).

### Blood Cell Preparation for Culture

Whole blood is collected by venipuncture into EDTA vacutainer tubes from healthy human donors. Peripheral blood mononuclear cells (PBMCs) are separated from whole blood by Histopaque®-1077 (Sigma Chemicals, St. Louis, MO) density gradient centrifugation. The PBMCs are suspended at 1.5-2 x 10⁶ cells/mL in RPMI 1640 medium containing 10 % fetal bovine serum, 2 mM L-glutamine and 1% penicillin/streptomycin solution (RPMI complete). 1 mL portions of PBMC suspension are added to 24 well flat bottom sterile tissue culture plates.

### Compound Preparation

The compounds are solubilized in dimethyl sulfoxide (DMSO). The DMSO concentration should not exceed a final concentration of 1% for addition to the culture wells. The compounds are generally tested in a concentration range of from 0.1 to 100 µM.

### Incubation

The solution of test compound is added to the wells containing 1 mL of PBMCs in media. The plates are covered with plastic lids, mixed gently and then incubated for 18 to 24 hours at 37°C with a 5% carbon dioxide atmosphere.

### Separation

Following incubation the plates are centrifuged for 5-10 minutes at 1000 rpm (~200 x g) at 4°C. The cell culture supernatant is removed with a sterile polypropylene pipet and transferred to a 2 mL sterile cryotube. Samples are maintained at -70°C until analysis.

### Interferon Analysis/Calculation

Interferon is determined by bioassay using A549 human lung carcinoma cells challenged with encephalomyocarditis. The details of the bioassay method have been described by G. L. Brennan and L. H. Kronenberg in "Automated Bioassay of Interferons in Micro-test Plates", Biotechniques, June/July, 78; 1983, incorporated herein by reference. Briefly stated the method is as follows: A549 cells are incubated with samples and standard interferon dilutions at 37°C for 24 hours. The incubated cells are then infected with an inoculum of encephalomyocarditis virus. The infected cells are incubated for an additional 24 hours at 37°C before quantifying for viral cytopathic effect. The viral cytopathic effect is quantified by staining followed by visual scoring of the plates. Results are expressed as alpha reference units/mL based on the value obtained for NIH Human Leukocyte IFN standard.

### Tumor Necrosis Factor (∞) Analysis

Tumor necrosis factor (∞) (TNF)concentration is determined using an ELISA kit available from Genzyme, Cambridge, MA. The results are expressed as pg/mL.

In the table below, a "+" indicates that the compound induced the indicated cytokine at that particular concentration, a "-" indicates that the compound did not induce the indicated cytokine at that particular concentration, and a "±" indicates that the results were equivocal at that particular concentration.

| **Cytokine Induction in Human Cells** | | | | | | | | |
|---|---|---|---|---|---|---|---|---|
| | IFN | | | | TNF | | | |
| Example | Dose Concentration (µM) | | | | Dose Concentration (µM) | | | |
| | 0.1 | 1.0 | 10.0 | 100.0 | 0.1 | 1.0 | 10.0 | 100.0 |
| 4 | Not run | + | + | + | Not run | - | - | - |

### INTERFERON (α) INDUCTION IN HUMAN CELLS

An *in vitro* human blood cell system was used to assess interferon induction by compounds of the invention. Activity is based on the measurement of interferon secreted into culture media. Interferon is measured by bioassay.

### Blood Cell Preparation for Culture

Whole blood was collected by venipuncture into EDTA vacutainer tubes. Peripheral blood mononuclear cells (PBM's) were separated from whole blood by using either LeucoPREP^{™} Brand Cell Separation Tubes (available from Becton Dickinson) or Ficoll-Paque® solution (available from Pharmacia LKB Biotechnology Inc, Piscataway, NJ). The PBM's were suspended at 1 x 10⁶/mL in RPMI 1640 media (available from GIBCO, Grand Island, NY) containing 25 mM HEPES (N-2-hydroxyethylpiperazine-N'-2-ethanesulfonic acid) and L-glutamine (1% penicillin-streptomycin solution added) with 10% heat inactivated (56°C for 30 minutes) autologous serum added. 200 µL portions of PBM suspension were added to 96 well (flat bottom) MicroTest III sterile tissue culture plates.

### Compound Preparation

The compounds were solubilized in ethanol, dimethyl sulfoxide or tissue culture water then diluted with tissue culture water, 0.01N sodium hydroxide or 0.01N hydrochloric acid (The choice of solvent will depend on the chemical characteristics of the compound being tested.). Ethanol or DMSO concentration should not exceed a final concentration of 1% for addition to the culture wells. Compounds were initially tested in a concentration range of from about 0.1 µg/mL to about 5 µg/mL. Compounds which show induction at a concentration of 0.5 µg/mL were then tested in a wider concentration range.

### Incubation

The solution of test compound was added in a volume (less than or equal to 50 µL) to the wells containing 200 µL of diluted whole blood or of PBM's in media. Solvent and/or media was added to control wells (wells with no test compound) and as needed to adjust the final volume of each well to 250 µL. The plates were covered with plastic lids, vortexed gently and then incubated for 48 hours at 37°C with a 5% carbon dioxide atmosphere.

### Separation

Following incubation, the plates were covered with parafilm and then centrifuged at 1000 rpm for 10 to 15 minutes at 4°C in a Damon IEC Model CRU-5000 centrifuge. Media (about 200 µL) was removed from 4 to 8 wells and pooled into 2 mL steri le freezing vials. Samples were maintained at -70°C until analysis.

### Interferon Analysis/Calculation

Interferon was determined by bioassay using A549 human lung carcinoma cells challenged with encephalomyocarditis. The details of the bioassay method have been described by G. L. Brennan and L. H. Kronenberg in "Automated Bioassay of Interferons in Micro-test Plates", Biotechniques, June/July, 78, 1983, incorporated herein by reference. Briefly stated the method is as follows: interferon dilutions and A549 cells are incubated at 37°C for 12 to 24 hours. The incubated cells are infected with an inoculum of encephalomyocarditis virus. The infected cells are incubated for an additional period at 37°C before quantifying for viral cytopathic effect. The viral cytopathic effect is quantified by staining followed by spectrophotometric absorbance measurements. Results are expressed as alpha reference units/mL based on the value obtained for NIH HU IF-L standard. The interferon was identified as essentially all interferon alpha by testing in checkerboard neutralization assays against rabbit anti-human interferon (beta) and goat anti-human interferon (alpha) using A549 cell monolayers challenged with encephalomyocarditis virus.

In the table below, a "+" indicates that the compound induced interferon α at that particular concentration, a "-" indicates that the compound did not induce interferon α at that particular concentration, and a "±" indicates that the results were equivocal at that particular concentration.

| Interferon (α) Induction in Human Cells | | | | | | | | | |
|---|---|---|---|---|---|---|---|---|---|
| Example | Dose Concentration (µg/mL) | | | | | | | | |
| | 0.01 | 0.05 | 0.10 | 0.50 | 1.0 | 5.0 | 10.0 | 25.0 | 50.0 |
| 4 | - | - | - | - | + | + | + | + | + |

The present invention has been described with reference to several embodiments thereof. The foregoing detailed description and examples have been provided fo r clarity of understanding only, and no unnecessary limitations are to be understood there from. It will be apparent to those skilled in the art that many changes can be made to the described embodiments without departing from the scope of the invention. Thus, the scope of the invention should not be limited to the exact details of the compositions and structures described herein, but rather by the language of the claims that follow.

## Claims

1. The compound 1-(2-methylpropyl)-1*H*-imidazo[4,5-*c*][1,5]naphthyridin-4-amine or a pharmaceutically acceptable salt thereof.

2. A pharmaceutical composition comprising a pharmaceutically effective amount of the compound or salt of claim 1 and a pharmaceutically acceptable carrier.

3. The compound or salt according to claim 1 for use in a method of inducing cytokine biosynthesis in an animal.

4. Use of the compound or salt according to claim 1 for preparing a medicament for inducing cytokine biosynthesis in an animal.

5. The compound or salt according to claim 1 for use in a method of treating a viral infection in an animal.

6. Use of the compound or salt according to claim 1 for preparing a medicament for treating a viral infection in an animal.

## Patentansprüche

1. Die Verbindung 1-(2-Methylpropyl)-1H-imidazo[4,5-c][1,5]naphthyridin-4-amin oder ein pharmazeutisch verträgliches Salz davon.

2. Arzneimittel, das eine pharmazeutisch wirksame Menge der Verbindung oder des Salzes nach Anspruch 1 und einen pharmazeutisch verträglichen Träger umfasst.

3. Verbindung oder Salz nach Anspruch 1 zur Verwendung bei einem Verfahren zur Induktion der Cytokin-Biosynthese in einem Tier.

4. Verwendung der Verbindung oder des Salzes nach Anspruch 1 zur Herstellung eines Arzneimittels zur Induktion der Cytokin-Biosynthese in einem Tier.

5. Verbindung oder Salz nach Anspruch 1 zur Verwendung bei einem Verfahren zur Behandlung einer Virusinfektion in einem Tier.

6. Verwendung der Verbindung oder des Salzes nach Anspruch 1 zur Herstellung eines Arzneimittels zur Behandlung einer Virusinfektion in einem Tier.

## Revendications

1. Composé, la 1-(2-méthylpropyl)-1*H*-imidazo[4,5-*c*]-[1,5]naphthyridin-4-amine, ou un sel pharmaceutiquement acceptable de celui-ci.

2. Composition pharmaceutique comprenant une quantité pharmaceutiquement efficace du composé ou sel selon la revendication 1 et un véhicule pharmaceutiquement acceptable.

3. Composé ou sel selon la revendication 1, pour une utilisation dans une méthode d'induction de la biosynthèse de cytokines chez un animal.

4. Utilisation du composé ou sel selon revendication 1, pour la préparation d'un médicament pour induire la biosynthèse de cytokines chez un animal.

5. Composé ou sel selon la revendication 1, pour une utilisation dans une méthode de traitement d'une infection virale chez un animal.

6. Utilisation du composé ou sel selon la revendication 1, pour la préparation d'un médicament pour le traitement d'une infection virale chez un animal.
